# FASCICULE DE BREVET EUROPEEN

(11) **EP 2 768 583 B1**
(45) Date de publication et mention de la délivrance du brevet: **06.09.2017**
(21) Numéro de dépôt: 12787787.6
(22) Date de dépôt: 19.10.2012
(51) Int. Cl.: A61K 8/34, A61Q 19/00, A61K 8/60, A61K 8/49, A61K 8/14

(54) **COMPOSITION COSMETIQUE OU DERMATOLOGIQUE COMPRENANT DES VESICULES DE POLYPENTOSIDE D'ALKYLE, ET SON PROCEDE DE PREPARATION.**
KOSMETISCHE ODER DERMATOLOGISCHE ZUSAMMENSETZUNG MIT ALKYL-POLYPENTOSIDE-VESIKELN UND VERFAHREN ZU IHRER HERSTELLUNG
COSMETIC OR DERMATOLOGICAL COMPOSITION COMPRISING ALKYL POLYPENTOSIDE VESICLES, AND METHOD FOR PREPARING SAME

(30) Priorité: 20.10.2011 FR 1159507
(43) Date de publication de la demande: 27.08.2014
(73) Titulaire: LVMH RECHERCHE, 45800 St. Jean de Braye (FR)
(72) Inventeur: PICHOT, Angélique, 45800 Saint Jean De Braye (FR); ALARD, Valérie, 45000 Orleans (FR); POUGET, Thierry, 45590 Saint Cyr En Val (FR); SCATTARELLI, Dominique, 45100 Orleans (FR); ERNENWEIN, Cédric, 02860 Nouvion Le Vineux (FR); ESTRINE, Boris, 51480 Nanteuil La Foret (FR)
(74) Mandataire: Chantraine, Sylvie Hélène
(86) Numéro de dépôt international: PCT/FR2012/052403
(87) Numéro de publication internationale: WO 2013/057455

(56) Documents cités:
- WO-A1-00/19980
- WO-A1-2005/110588
- WO-A1-2008/135646
- DE-A1- 19 634 374
- FR-A1- 2 945 208
- US-A1- 2005 136 081
- KIWADA H ET AL: "application of synthetic alkyl glycoside vesicles as drug carriers. I. preparation and physical properties", CHEMICAL & PHARMACEUTICAL BULLETIN, PHARMACEUTICAL SOCIETY OF JAPAN, JP, vol. 33, no. 2, 1 février 1985 (1985-02-01), pages 753-759, XP002129540, ISSN: 0009-2363

## Description

La présente invention a pour objet une composition cosmétique ou dermatologique comprenant un alcool, un agent tensioactif et un polypentoside d'alkyle. L'invention concerne également un procédé de préparation d'une telle composition.

Les polyglycosides d'alkyle sont des molécules amphiphiles formés d'une partie hydrophile constituée d'un ose greffée à une partie lipophile constituée d'un radical alkyle. Ces composés peuvent former, en phase aqueuse et dans certaines conditions, des vésicules.

Le document DE 19634374-A1 décrit des dispersions de vésicules formées avec des polyglycosides d'alkyle (en abrégé APG) de formule (1):

RO(G)ₙ (1)

dans laquelle R est un radical aliphatique linéaire ayant de 12 à 22 atomes de carbone, G représente un ose ou un oligoside, et le degré de polymérisation n du sucre est compris entre 1 et 2. Ces vésicules sont préparés à partir du mélange de l'ose ou de l'oligoside (produit A) avec un alcool gras linéaire de 12 à 22 atomes de carbone (produit B) dans un rapport massique A/B allant de 10/1 à 1/2. L'ose est de préférence le glucose.

La demande de brevet française FR 10 01755 déposée le 23 avril 2010 et non publiée à ce jour, divulgue les polypentosides d'alkyle de formule (2) :

RO(X)ₙ (2)

dans laquelle R est un radical alkyle aliphatique linéaire ou ramifié, avec ou sans insaturation, X est un xylose et n est compris entre 1 et 1,5. Selon l'enseignement de ce document, ces polyglycosides d'alkyle présentent l'avantage de pouvoir former des vésicules dans des compositions dont la phase continue est constituée d'eau, mais aussi dans des compositions dont la phase continue est une phase grasse.

Or la Demanderesse a pu constater que certains polypentosides d'alkyle présentent l'inconvénient de cristalliser au cours du temps quand ils sont dispersés dans des compositions cosmétiques ou dermatologiques à phase continue aqueuse.

La Demanderesse a pu également constater que la formule de vésicules formées à partir de certains polypentosides d'alkyle n'est pas stable au cours du temps lors de leur incorporation dans une composition cosmétique par formulation dans un mélange d'huile, d'eau et de gélifiant mais aussi par simple dilution dans l'eau. Or le maintien de l'intégrité de la structure des vésicules est indispensable à la fois pour conserver la texture et les propriétés organoleptiques de la composition, et pour conserver les actifs éventuels que les vésicules véhiculent.

La formulation des compositions à phase continue aqueuse doit donc être optimisée pour empêcher la cristallisation des polypentosides d'alkyle et maintenir l'intégrité des vésicules, tout en conservant les conditions qui permettent aux polypentosides d'alkyle de former des vésicules dans la phase aqueuse.

Les inventeurs ont mis en évidence qu'en milieu aqueux, il est indispensable de sélectionner la nature des polypentosides d'alkyle aptes à former des vésicules et de leur adjoindre des composés de façon à éviter toute instabilité au cours du temps, se traduisant par exemple par la cristallisation de polypentosides.

Ce résultat a été obtenu à l'aide de la composition de l'invention qui comprend, outre un polypentoside d'alkyle particulier, au moins un tensioactif particulier et au moins un solvant particulier.

Il a été mis en évidence que pour obtenir une composition stable dont la phase continue est aqueuse et qui comprend des vésicules à base de polypentosides d'alkyle, il est nécessaire d'ajouter à la phase continue aqueuse au moins un tensioactif de HLB inférieur de 10, ainsi qu'un alcool comprenant de 1 à 5 atomes de carbone.

Les inventeurs ont en outre démontré que les compositions de l'invention, une fois appliquées sur la peau, permettent d'améliorer significativement la pénétration cutanée d'agents actifs encapsulés dans les vésicules.

La présente invention a ainsi pour objet une composition cosmétique ou dermatologique comprenant au moins un agent actif cosmétique ou dermatologique et au moins une phase aqueuse continue dans laquelle sont dispersées des vésicules,
- lesdites vésicules comprenant au moins un polypentoside d'alkyle obtenu par réaction d'un pentose et d'au moins un premier et un deuxième alcools gras comprenant indépendamment l'un de l'autre un nombre d'atomes de carbone allant de 8 à 12, chacun de ces alcools gras pouvant être saturé ou insaturé, linéaire ou ramifié, ledit polypentoside ayant un degré de polymérisation moyen inférieur ou égal à 2, et
- ladite composition comprenant en outre au moins un tensioactif de HLB inférieur à 10, et au moins un alcool ayant de 1 à 5 atomes de carbone.

Les molécules amphiphiles - en particulier les polyglycosides d'alkyle - qui sont formées d'une tête hydrophile reliée à un groupement lipophile - s'organisent en bicouche (ou double couche) en présence d'eau.

On entend par « vésicules » des agrégats supra moléculaires essentiellement sphériques, de taille micronique ou sub-micronique (généralement de 0,1 à 10 µm), constitués de plusieurs couches de molécules amphiphiles. On parle aussi parfois de « liposome » par analogie de structure, bien que les molécules amphiphiles qui constituent les vésicules de l'invention ne soient pas des lipides.

Les vésicules peuvent être multilamellaires (MLV en abrégé) quand elles sont constituées d'un empilement de plusieurs double-couches de molécules amphiphiles, ou unilamellaires (en abrégé ULV) quand elles sont constituées d'une seule double-couche. Les vésicules multilamellaires comprennent à l'intérieur de leur structure plusieurs compartiments aqueux, localisés entre les bicouches et au coeur, tandis que les vésicules unilamellaires en contiennent un seul en leur centre.

Les vésicules de l'invention sont avantageusement multilamellaires. On peut l'observer par microscopie électronique après cryofracture.

On entend par vésicule "stable", une vésicule dont la taille moyenne au cours d'un stockage à une température comprise entre 4°C et 50°C, pendant une durée d'au moins 30 jours, de préférence d'au moins 90 jours à 4°C, est constante.

On entend par "diluable", la possibilité de diluer les vésicules dans l'eau à un facteur de dilution allant de 1 à 30 fois, et de préférence allant de 2 à 100 fois sans modifier substantiellement la forme et la taille des vésicules.

Le pentose peut être choisi parmi le xylose, l'arabinose, le ribose ou le xylulose, sous sa forme isomérique alpha ou bêta, de la série L ou D, et sous sa forme furanosique ou pyranosique.

Selon un mode de mise en oeuvre, le pentose est le xylose, sous forme isomérique alpha ou bêta, de la série L ou D. On préfère le D-xylose.

Le degré de polymérisation moyen du pentose est inférieur ou égal à 2, de préférence compris entre 1 et 1,8, de préférence encore compris entre 1,3 et 1,6, et plus préférentiellement allant de 1,4 à 1,6, et avantageusement environ égal à 1,5.

Le polypentoside d'alkyle est obtenu par réaction d'un pentose et d'au moins un premier et un deuxième alcool gras comprenant indépendamment l'un de l'autre un nombre d'atomes de carbone allant de 8 à 12, de préférence allant de 8 à 10. On entend par « alcool gras » un alcool comprenant de 8 à 12 atomes de carbones, ledit alcool étant aliphatique (i.e. non aromatique ne comprenant pas de doubles liaisons carbone-carbone conjuguées procurant une aromaticité) et comprenant une fonction -OH.

Le premier et le deuxième alcool gras sont différents. Ils ont de préférence un nombre d'atomes de carbone différent. Les alcools gras sont de préférence tous deux linéaires et saturés.

Le premier alcool gras est saturé ou insaturé, linéaire ou ramifié, et comprend un nombre d'atomes de carbone allant de 10 à 12. Le premier alcool gras est de préférence linéaire saturé et comprend par exemple 10, 11 ou 12 atomes de carbone, de préférence 10 atomes de carbone. On préfère le décanol-1.

Le deuxième alcool gras est de préférence saturé ou insaturé, linéaire ou ramifié, et comprend un nombre d'atomes de carbone allant de 8 à 9. Il est de préférence linéaire saturé et comprend 8 atomes de carbone. On préfère l'octanol-1.

Selon un mode de mise en oeuvre, le premier alcool gras est linéaire saturé et comprend de 10 à 12 atomes de carbone, et/ou le deuxième alcool gras est linéaire saturé et comprend 8 ou 9 atomes de carbone. De préférence, Le premier alcool gras est le décanol-1 et/ou le deuxième alcool gras est l'octanol-1.

Le rapport massique entre le premier et le deuxième alcool gras est avantageusement compris entre 80/20 et 99/1, de préférence compris entre 85/15 et 95/5, notamment de l'ordre de 90/10.

Un polypentoside d'alkyle préféré est obtenu à partir du D-xylose, du décanol-1 et de l'octanol-1, et le ratio entre le décanol-1 et l'octanol-1 est compris entre 80/20 et 99/1, de préférence compris entre 85/15 et 95/5, de manière encore préférée environ égal à 90/10.

La composition de l'invention comprend avantageusement de 0,01 à 20% en poids total de polypentoside d'alkyle, de préférence de 0,1 à 10% en poids de polypentoside d'alkyle, et de manière encore préférée de 0,1 à 5% en poids de polypentoside d'alkyle.

L'alcool comprend avantageusement de 1 à 5 atomes de carbone ; il est de préférence aliphatique (i.e. cyclique ou acyclique, saturé ou insaturé, et non aromatique), et choisi parmi les mono-alcools et les diols.

On choisit de préférence le mono-alcool parmi l'éthanol, le butanol-1 et le pentanol-1. Le diol peut être le 2,3-butylène glycol ou le propane-1,2-diol (encore appelé propylène glycol).

La composition comprend avantageusement de 0,01 à 5% en poids, et de préférence de 0,1 à 1% en poids d'alcool.

On préfère un rapport massique [polypentoside d'alkyle/alcool] compris entre 1 et 9, de préférence compris entre 6 et 8.

Le tensioactif a une valeur d'HLB avantageusement comprise entre au moins 5 et 10. La valeur de la Balance-Hydrophile-Lipophile (HLB) d'un tensioactif non ionique peut être obtenus par calcul selon la méthode de Griffin (Griffin WC: "Calculation of HLB Values of Non-Ionic Surfactants", Journal of the Society of Cosmetic Chemists 5 (1954): 259).

Le tensioactif est avantageusement un tensioactif non-ionique.

On préfère comme tensioactif d'HLB inférieur ou égal à 10 les esters de sorbitan et d'un acide gras aliphatique non hydroxylé comme le monolaurate de sorbitan, le mono-oléate de sorbitan, le mono-stéarate de sorbitan, le tri-oléate de sorbitan, le tri-stéarate de sorbitan, le sesquioléate de sorbitan, et les esters de sorbitan faiblement éthoxylés comme le POE(2) stéarate de sorbitan.

Par acide gras aliphatique on entend un acide carboxylique, de préférence monocarboxylique, de préférence aliphatique (non aromatique), cyclique ou acyclique, saturé ou insaturé, linéaire ou ramifié. On préfère un acide monocarboxylique linéaire et saturé.

Selon un mode de mise en oeuvre préféré, on utilise le monolaurate de sorbitan.

La composition comprend avantageusement de 0,01 à 6% en poids, et de préférence de 0,1 à 2% en poids dudit tensioactif.

On préfère ainsi un rapport massique [polypentoside d'alkyle/tensioactif] compris entre 1 et 5, de préférence compris entre 2 et 4.

Selon une mise en oeuvre particulièrement préférée de l'invention, la composition comprend du monolaurate de sorbitan, de l'éthanol et un polypentoside d'alkyle obtenu par réaction du D-xylose, du 1-décanol et du 1-octanol, ledit polypentoside ayant un degré de polymérisation moyen compris allant de 1,4 et 1,6.

Selon une mise en oeuvre préférée, le rapport massique [polypentoside d'alkyle/éthanol] est compris entre 6 et 8.

Selon une autre mise en oeuvre préférée, le rapport massique [polypentoside d'alkyle/monolaurate de sorbitan] est compris entre 2 et 4.

Les inventeurs ont mis en évidence qu'une composition selon l'invention permet d'améliorer significativement la pénétration cutanée d'agents actifs encapsulés dans les vésicules dispersées dans la composition, une fois que celle-ci a été appliquée sur la peau du corps ou du visage.

Ainsi l'invention est particulièrement utile dans le domaine de la cosmétique et de la dermatologie afin d'encapsuler des agents actifs.

L'agent actif cosmétique ou dermatologique est ainsi au moins partiellement encapsulé dans les vésicules dispersées dans la phase aqueuse de la composition de l'invention.

Ces vésicules peuvent contenir dans les doubles couches, entre les doubles couches et/ou dans leurs coeurs une phase aqueuse ou non aqueuse pouvant contenir un ou plusieurs agents actifs solubilisés ou dispersés.

La composition de l'invention comprend au moins un agent actif cosmétique ou dermatologique, lequel peut être sous forme de molécules purifiées et/ou d'extraits, notamment d'extraits végétaux, présentant des effets cosmétiques ou dermatologiques.

L'agent cosmétique ou dermatologique peut être avantageusement choisi parmi les composés ayant une activité anti-vieillissement, pour prévenir ou retarder l'apparition des signes du vieillissement cutané ou pour en ralentir ou atténuer les effets, ou bien encore pour favoriser la longévité cellulaire ou tissulaire; ceux ayant une activité dépigmentante, blanchissante ou éclaircissante de la peau; ceux ayant une activité amincissante; ceux ayant une activité hydratante; ceux ayant une activité calmante, apaisante, relaxante ou anti-inflammatoire; ceux ayant une activité stimulant la microcirculation cutanée pour améliorer l'éclat du teint, en particulier du visage; ceux ayant une activité sébo-régulatrice pour le soin des peaux grasses; ceux destinées à nettoyer ou purifier la peau; ceux ayant une activité anti-radicalaire, et l'un quelconque de leur mélanges. Les actifs peuvent être par exemple choisis parmi :
- des vitamines, comme la vitamine A, E ou C,
- des anti-inflammatoires comme des extraits de plantes, l'alpha-bisabolol, le panthénol, l'alpha-tocophérol,
- des anti-âges comme le rétinol, l'adénosine,
- des agents autobronzants comme la dihydroxyacétone (DHA), l'érythrulose,
- des agents dépigmentants comme l'acide kojique, l'acide coumarique ou l'arbutine,
- des actifs amincissants comme la caféine,
- des filtres UV comme les dérivés de la benzophénone, les esters d'acides cinnamiques, les esters d'acide salicylique, le 3-benzylidène camphre, les dérivés de triazine,
- des antioxydants comme l'acide ascorbique et ses dérivés, l'acide citrique et ses dérivés, l'acide glutamique, les glutamates et ses dérivés, l'acide lactique et ses dérivés, l'acide tartrique et ses dérivés, les bioflavonoïdes, le butylhydroxy-hydroxyanisol, le carotène et ses dérivés, les sulfites comme les bisulfites de sodium, le chlorobutanol,
- les agents hydratants comme le glycérol, le sorbitol, le collagène, le pro-collagène, la gélatine, l'aloe-vera, l'acide hyaluronique, l'urée, propanediol, butylène glycol, diglycérine.

La composition à phase aqueuse continue de l'invention peut être sous la forme d'une solution aqueuse ou hydro-alcoolique, d'une dispersion, d'un gel aqueux ou hydro-alcoolique, ou encore d'une émulsion huile dans eau (H/E), destinée à être appliquée sur la peau du corps ou du visage.

Elle peut se présenter sous la forme de crèmes, de laits, de pommades, d'onguents, de gels ou de lotions, ou encore de produits de maquillage.

La composition peut en outre contenir un agent gélifiant de la phase aqueuse. On entend par agent gélifiant de la phase aqueuse, un composé qui en augmente la viscosité.

Les agents gélifiants incluent, mais ne sont pas limités, aux polymères d'origine naturelle tels que la gomme de caroube, l'alginate de sodium, l'agar, la gomme xanthane (Rhodicare XC), les amidons, les dérivés de cellulose (par exemple hydroxyéthylcellulose, méthylcellulose, carboxyméthylcellulose, hydroxypropylmethyl cellulose), les polymères carboxyvinyliques, la polyvinylpyrrolidone, l'alcool polyvinylique polymères acryliques, les polymères d'acide méthacrylique, les polymères d'acide d'acétate de polyvinyle, les polymères de chlorure de vinyle, de vinylidène polymères du chlorure et analogues. Éventuellement, des mélanges des composés ci-dessus sont envisagés.

Sont également utiles des agents gélifiants tels que i) les polymères de l'acide acrylique réticulés, notamment les copolymères de l'acide acrylique et de l'acrylate d'alkyle réticulé avec un éther d'allyle de pentaétythitol ou de saccharose (Acrylate/C10-C30 alkylacrylate crosspolymer), tels que ceux vendus sous la marque Pemulene (TR1 ou TR2), et ii) les polymères carboxyvinyliques tels que ceux vendus sous la marque Carbopol.

L'agent gélifiant est éventuellement présent dans la composition en une quantité d'environ 0,1% à 5,0% en poids du poids de la composition. Dans une forme de réalisation, l'agent gélifiant représente environ 1% en poids de la composition cosmétique.

Avantageusement, la composition de l'invention comprend en outre au moins un excipient acceptable d'un point de vue cosmétique ou dermatologique pouvant être choisi parmi des pigments, des colorants, des polymères, des agents tensioactifs, des agents de rhéologie, des parfums, des électrolytes, des ajusteurs de pH, des agents anti-oxydants, des conservateurs, et l'un quelconque de leurs mélanges.

Un autre objet de l'invention vise un procédé de préparation de la composition de l'invention comprenant les étapes suivantes :
- la préparation d'une phase aqueuse comprenant avantageusement l'agent actif cosmétique ou dermatologique qu'on souhaite encapsuler au moins partiellement dans les vésicules, suivie de,
- la dispersion du polypentoside d'alkyle dans ladite phase aqueuse, en apportant de l'énergie notamment sous la forme d'un cisaillement permettant la formation de vésicules à la température ambiante ou à une température supérieure mais de préférence inférieure à 100°C, de préférence inférieure à 80°C,
ledit procédé étant caractérisé en ce que l'alcool et l'agent tensioactif d'HLB inférieur à 10 tels que décrits précédemment sont chacun ajoutés partiellement ou en totalité, indifféremment soit dans la phase aqueuse, soit en mélange avec le polypentoside d'alkyle pour être dispersés dans ladite phase aqueuse.

On choisit de préférence des mélangeurs à bas cisaillements comme des agitateurs pendulaires munis d'une ou plusieurs hélices ou d'une ancre, des agitateurs à double rotations munis d'une agitation centrale à une ou plusieurs hélices et d'une agitation périphérique munis d'une ou plusieurs pâles raclantes épousant la forme du réacteur ou bien encore des pétrins et malaxeurs. La vitesse de rotation de ces mélangeurs est de préférence supérieure ou égale à 500 tours/minutes.

Un autre objet de la présente invention vise une méthode de soin cosmétique comprenant l'application sur la peau d'au moins une partie du corps ou du visage, d'une composition telle que décrite précédemment notamment pour améliorer la pénétration cutanée d'au moins un agent actif cosmétique ou dermatologique au moins partiellement encapsulé dans des vésicules dispersées dans la phase continue aqueuse de ladite composition.

L'invention concerne également la composition décrite précédemment pour son utilisation dans le traitement dermatologique des maladies de la peau.

Selon encore un autre objet, l'invention concerne l'utilisation de la combinaison i) de vésicules comprenant au moins un polypentoside d'alkyle obtenu par réaction d'un pentose et d'au moins un premier et un deuxième alcools gras comprenant indépendamment l'un de l'autre un nombre d'atomes de carbone allant de 8 à 12, chacun de ces alcools gras pouvant être saturé ou insaturé, linéaire ou ramifié, ledit polypentoside ayant un degré de polymérisation moyen inférieur ou égal à 2, et ii) d'au moins un tensioactif de HLB inférieur à 10, et iii) d'au moins un alcool ayant de 1 à 5 atomes de carbone,
pour améliorer la pénétration cutanée d'au moins un agent actif cosmétique ou dermatologique.

L'invention a encore pour objet l'utilisation de l'association d'un alcool ayant de 1 à 5 atomes de carbone et d'un agent tensio-actif de HLB inférieur à 10 pour stabiliser des vésicules formées à partir de polypentosides comprenant au moins un polypentoside d'alkyle obtenu par réaction d'un pentose et d'au moins un premier et un deuxième alcools gras comprenant indépendamment l'un de l'autre un nombre d'atomes de carbone allant de 8 à 12, ledit polypentoside ayant un degré de polymérisation moyen inférieur ou égal à 2, dans la phase aqueuse d'une composition.

L'association de l'alcool et du tensio-actif permet avantageusement de stabiliser les vésicules pour éviter toute cristallisation des polypentosides, et pour que la taille et la forme de vésicules restent constante au cours du temps dans la composition cosmétique ou dermatologique.

L'alcool et l'agent tensio-actif sont tels que décrits précédemment. L'alcool est de préférence un mono-alcool ou un diol, avantageusement l'éthanol.

L'agent tensio-actif est de préférence un ester de sorbitan et d'un acide gras aliphatique non hydroxylé, avantageusement le monolaurate de sorbitan.

Selon une mise en oeuvre particulièrement préférée, on utilise ainsi l'éthanol et un ester de sorbitan et d'un alcool gras non hydroxylé, avantageusement le monolaurate de sorbitan, pour stabiliser des vésicules formées à partir d'au moins un polypentoside d'alkyle obtenu par réaction du D-xylose, et d'au moins deux alcools gras comprenant un nombre d'atomes de carbone allant de 8 à 12, avantageusement le 1-décanol et le 1-octanol, ledit polypentoside ayant un degré de polymérisation moyen inférieur ou égal à 2, avantageusement compris entre 1,4 et 1,6.

Les caractéristiques qui ont été décrites en rapport avec la composition sont valables pour décrire le procédé de préparation et l'utilisation, objets de la présente invention.

L'invention est illustrée plus en détail par les exemples suivants.

### Exemple 1 : Composition aqueuse selon l'invention

### Préparation

On a préparé un polyxylose d'octyle et de décyle mettant en oeuvre le D-xylose et un mélange d'alcool gras en excès selon un procédé connu de l'homme du métier tel que celui décrit aux exemples 1 et 2 de la demande de brevet EP 1750834 (AGRO INDUSTRIE RECHERCHES ET DEVELOPPEMENTS).

On a mis en contact en milieu acide du D-Xylose et un mélange d'alcools gras comprenant le 1-décanol (premier alcool gras, C10) et le 1-octanol (deuxième alcool gras, C8), dans un rapport massique [C10/C8] de 90/10.

Le polyxylose obtenu - noté Polyxylose d'alkyle (C8/C10) - est utilisé dans la composition du présent exemple ainsi que dans les exemples comparatifs 2.1 à 2.3.

Les conditions de préparation ont été adaptées de façon à ce que le polyxylose d'octyle et de décyle présente un degré de polymérisation d'environ 1,5.

On a préparé une composition sous la forme d'un gel aqueux de composition suivante (les pourcentages sont en poids).

| Phase A | |
|---|---|
| Caféïne | 1,0 |
| Phénoxyéthanol | 1,0 |
| Eau purifiée | qsp 100 |

| Phase B | |
|---|---|
| Sodium Acrylates Copolymer | 1,0 |
| Polyisobutène hydrogéné | 0,75 |
| Phospholipides | 0,25 |
| Polyglycéryl-10 Stéarate | 0,25 |
| Huile de graines de tournesol (Helianthus Annuus) | 0,25 |
| Tocophéryl acétate | < 0,01 |

| Phase C | |
|---|---|
| Polyxylose d'alkyle (C8/C10) | 3,5 |
| Monolaurate de sorbitan | 1,0 |
| Ethanol | 0,5 |

On a préparé la phase A et la phase B en mélangeant les ingrédients qui les composent. Les phases A et B ont été mélangées jusqu'à obtenir un mélange homogène. On a ensuite ajouté à ce mélange les composés de la phase C, et appliqué un cisaillement au mélangeur sur potence Rayneri^{®} équipé d'une pale défloculeuse à une vitesse de 500 tr/min pendant 15 minutes permettant la formation de vésicules multilamellaires. Après homogénéisation, les vésicules formées sont dispersées dans la phase continue de la composition ainsi préparée.

### Caractérisation des vésicules

Juste après la préparation de la composition, on a observé la présence de vésicules par microscopie électronique après congélation et cryofracture d'un échantillon de la composition.

La méthode qui a été utilisée est le cryodécapage qui permet de réaliser une réplique de la structure observable par microscopie électronique à transmission. Cette technique comporte quatre étapes essentielles:
1 la congélation ;
2 la fracture et le "etching" ;
3 l'ombrage et la formation de la réplique ;
4 le nettoyage de la réplique.

Enfin la visualisation de la réplique à l'aide d'un microscope électronique à transmission et l'analyse visuelle des clichés.

Les clichés montrent une dispersion homogène d'objets sphériques ou quasi-sphériques en positif et négatif et présentant dans le cas des vésicules multilamellaires plusieurs stries sphériques en périphérie traduisant l'enroulement de plusieurs couches.

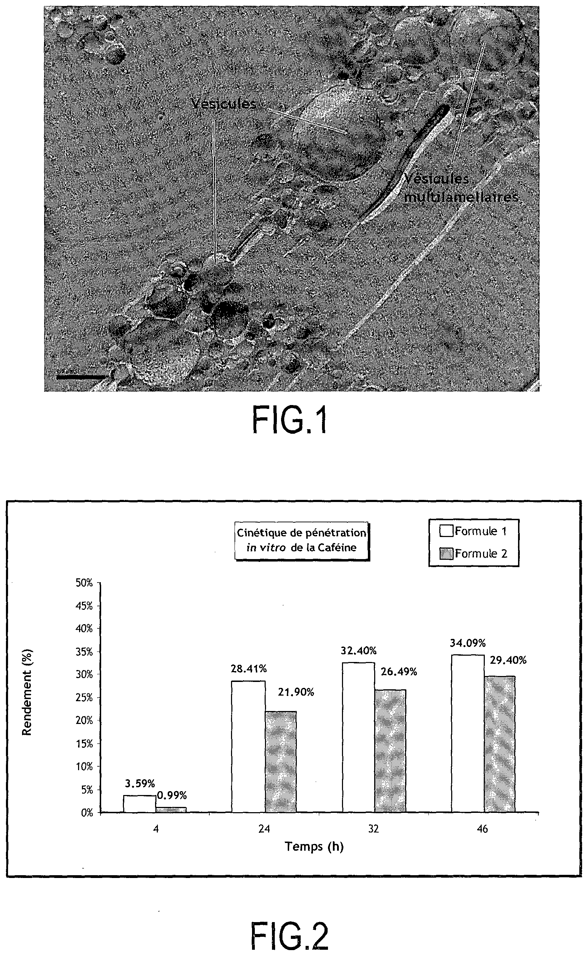

La figure 1 est une photographie montrant une dispersion d'objets sphériques dans le milieu de la composition, caractéristiques de la présence de vésicules multilamellaires.

### Etude de stabilité au cours du temps

On a soumis des échantillons de chacune la composition de l'invention à différentes conditions de luminosité et de température de façon à tester sa stabilité.

**Stabilité de la composition de l'exemple 1 tableau 1)**

| | 4°C | 45°C | Obscurité (température ambiante) |
|---|---|---|---|
| T0 | gel jaune pâle, odeur caractéristique | | |
| 1 mois | Gel stable | Gel stable | Gel stable |
| 1,5 mois | | | présence de vésicules dans le gel (fig. 1) |
| 15 mois | gel blanc crème, stable | couleur jaunâtre, fluide avec léger relargage d'eau en surface | Gel stable |

On a stocké la composition à 4°C, à 25°C et 45°C pendant quinze mois puis on l'a observée visuellement et au microscope optique (grossissement x10).

Des répliques de cryofractures ont été réalisées et observées par microscopie électronique à transmission sur des échantillons n'ayant pas été stockés et sur des échantillons qui ont été jugés stables à température ambiante et obscurité au bout de 45 jours (1,5 mois), selon la méthode décrite précédemment.

L'observation sous microscope optique a permis de vérifier la présence de vésicules dans la composition stockée après 15 mois de stockage dans ces conditions.

Dans les conditions de test, la composition de l'invention est aussi stable que la formule témoin exempte de phase C formant les vésicules (exemple comparatif 2.4, tableau 2). L'ajout de la phase C formant les vésicules dans la composition n'induit aucun phénomène défavorable vis-à-vis de la stabilité.

Au final, on observe que la préparation d'une composition stable au cours du temps, qui comprend des vésicules à base de polypentoside d'alkyle, n'est possible qu'en présence dans la formule d'un alcool (éthanol dans le présent exemple) et d'un tensio-actif de HLB inférieur à 10 (monolaurate de sorbitan dans le présent exemple).

### Stabilité du témoin sans vésicules

On a préparé la composition de formule ci-dessous, qui diffère de la composition précédente en ce qu'elle ne comprend pas les composés de la phase C, et qui est donc exempte de vésicules (les pourcentages sont exprimés en poids).

| Phase A | |
|---|---|
| Caféine | 1,0 |
| Phénoxyéthanol | 1,0 |
| Eau | qsp 100 |

| Phase B | |
|---|---|
| Sodium Acrylates Copolymer | 1,0 |
| Polyisobutène hydrogéné | 0,75 |
| Phospholipides | 0,25 |
| Polyglycéryl-10 Stéarate | 0,25 |
| Huile de graines de tournesol (*Helianthus Annuus*) | 0,25 |
| Tocophéryl acétate | < 0,01 |

On a soumis des échantillons la formule témoin à différentes conditions de luminosité et de température de façon à en évaluer la stabilité.

**Stabilité de la Formule témoin sans vésicules (tableau 2)**

| | 4°C | 45°C | Obscurité (température ambiante) |
|---|---|---|---|
| T0 | gel jaune clair, odeur caractéristique | | |
| 14 mois | gel blanc, stable | gel blanc crème, très léger relargage en surface, stable | gel jaune clair, stable |

Après 14 mois dans les conditions décrites dans le tableau, la formule témoin ne présente pas de modification significative de sa texture. La composition est stable.

### Exemple 2 : Etude de stabilité

### Exemple comparatif 2.1

On a préparé une composition aqueuse de formule ci-dessous. Les pourcentages sont en poids.

| Phase A | |
|---|---|
| Caféine | 1,0 |
| Eau | qs 100 |

| Phase B | |
|---|---|
| Polyxylose d'alkyle C8/C10 de l'exemple 1 | 15,0 |

On a dispersé le polyxylose d'alkyle préparé selon le procédé décrit dans l'exemple 1 dans la solution de caféine précédemment préparée.

On a vérifié par microscopie optique la présence de vésicules.

La composition a été mise d'une part à l'obscurité à température ambiante et d'autre part à l'étuve à 4°C.

Le résultat obtenu est le suivant :
- après 1 mois à 4°C : apparition de cristaux dans la composition.
- après 3 mois à température ambiante : apparition de cristaux dans la composition.

La composition préparée dans cet exemple comparatif, qui ne contient ni alcool ni tensioactif de HLB inférieur à 10 n'est pas stable dans les conditions d'étude.

### Exemple comparatif 2.2

On a préparé une composition aqueuse de formule ci-dessous, qui se distingue de celle de l'invention en ce qu'elle ne contient aucun alcool. Les pourcentages sont en poids.

| Phase A | |
|---|---|
| Caféine | 1,0 |
| Eau | qs 100 |

| Phase B | |
|---|---|
| Polyxylose d'alkyle C8/C10 de l'exemple 1 | 15,0 |
| Monolaurate de sorbitan | 1,0 |

On a dispersé les composés de la phase B dans la solution de caféine (phase A) précédemment préparée.

On a vérifié par microscopie optique la présence de vésicules dans la composition cosmétique.

La composition a été conservée d'une part à l'obscurité à température ambiante et d'autre part à l'étuve à 4°C.

Le résultat obtenu est le suivant :
- après 1 mois à 4°C : apparition de cristaux dans la composition.
- après 3 mois à température ambiante : apparition de cristaux dans la composition.

En absence d'un alcool, la composition préparée dans cet exemple comparatif, n'est pas stable dans les conditions d'étude.

Selon une variante de cet exemple comparatif, on a remplacé le monolaurate de sorbitan par le sesquioleate de sorbitan (HLB=3,7).

On a observé également l'apparition de cristaux dans la composition au cours de l'étude de stabilité.

### Exemple comparatif 2.3

On a préparé une composition aqueuse de formule ci-dessous, qui contrairement à celle de l'invention, ne comprend aucun tensioactif de HLB inférieur à 10.

| Phase A | |
|---|---|
| Caféine | 1,0 |
| Eau | qs 100 |
| Acrylates/C10-30 Alkyl Acrylate Crosspolymer | 0,4 |

| Phase B | |
|---|---|
| Polyxylose d'alkyle C8/C10 de l'exemple 1 | 4,75 |
| Ethanol | 0,25 |

On a préparé la phase A en dispersant les polymères dans l'eau.

On a ajouté la phase B, puis on a homogénéisé la composition aqueuse par cisaillement léger.

On a observé la présence de phases lamellaires non vésiculaires.

En l'absence de co-tensioactif de HLB inférieur à 10, on n'observe aucune vésicule formée dans la phase aqueuse, contrairement à ce qui est observé pour la composition de l'invention.

Les résultats sont résumés dans le tableau ci-dessous.

| INGREDIENTS | Exemple 2 | Exemple comparatif 2.1 | Exemple comparatif 2.2 | Exemple comparatif 2.3 |
|---|---|---|---|---|
| Caféine | 1,0 | | | |
| Eau | qs 100 | | | |
| Acrylates/C10-30 Alkyl Acrylate Crospolymer | 0,4 | - | - | 0,4 |
| Polyxylose d'alkyle C8/C10 de l'exemple 1 | 14,75 | 15,0 | 15,0 | 14,75 |
| Monolaurate de sorbitan | 1,0 | - | 1,0 | - |
| Ethanol | 0,25 | - | - | 0,25 |

| STABILITE | | | | |
|---|---|---|---|---|
| après 1 mois à 4°C | stable | cristaux | cristaux | Pas de vésicules |
| après 3 mois à 20°C | stable | cristaux | cristaux | Pas de vésicules |

### Exemple 3 : Etude de pénétration cutanée

### But de l'étude

Les actifs hydrophiles franchissent difficilement le *stratum corneum,* barrière cutanée plus lipophile, d'où l'intérêt de les vectoriser pour en favoriser la pénétration.

Le but de l'étude, réalisée *in vitro* sur peau d'oreille de porc totale congelée, était d'évaluer l'efficacité des vésicules à base de polypentosides d'alkyle, pour améliorer la pénétration et la distribution au sein des couches superficielles de la peau d'actifs distribués inégalement, notamment du fait de leur hydrophilie/amphiphilie.

### Principe

On a mesuré la diffusion d'un traceur, la caféine, à travers la peau d'oreille de porc décongelée, montée sur une cellule de diffusion de Franz, en conditions occlusives.

La caféine a été choisie comme traceur du fait de son hydrophilie qui la rend peu apte à passer la barrière cutanée.

Une cellule de diffusion de Franz comprend deux compartiments superposés communiquant au travers de la membrane utilisée pour l'étude.

La peau, utilisée comme membrane, est placée *stratum corneum* orienté vers le haut, entre ces deux compartiments. La solution aqueuse ou la composition galénique à tester contenant de la caféine est introduite dans le compartiment supérieur au contact de la peau. Une certaine quantité de caféine, dissoute dans la solution ou la composition, traverse la membrane constituée par la peau, puis est recueillie au niveau du compartiment inférieur dans une solution dite réceptrice. L'appareil de prélèvement recueille un échantillon de solution réceptrice à intervalles de temps réguliers. Les échantillons sont dosés en HPLC afin de déterminer la quantité de caféine ayant traversé la peau. Le traitement des données permet le calcul des flux de caféine, les cinétiques de pénétration sur 40h ainsi que les rendements d'absorption en 24h.

### Conditions de réalisation

### - Quantité et mode d'application :

Après la décongélation et la sélection des peaux, des zones de dépôt ont été déterminées et limitées sur chacune de ces peaux afin qu'elles aient une surface de 9 cm². Ensuite, chaque composition a été déposée, à raison de 2 mg/cm², à l'aide d'une micropipette et appliquée en utilisant un doigtier préalablement saturé par les galéniques.

La cinétique de pénétration a été établie par prélèvements de 0.2 ml du liquide récepteur à 4h, 8h, 24h 28h, 32h et 46h, à l'aide d'une pipette à prélèvement manuelle. Les échantillons prélevés ont été dosés par HPLC.

La pénétration cutanée des actifs était, dans le cadre de cette étude, principalement utilisée à titre comparatif. Différentes formulations ont été évaluées par cette technique dans le but de mettre en évidence celles qui favorisent ou limitent la pénétration de divers actifs.
- Formule testées : composition de l'exemple 1 et formule témoin sans vésicules conforme à l'exemple comparatif 2.4.
- Paramètres de l'étude :

### - Cellule de Franz « Lara Spiral » :

Surface d'exposition de 3.8 cm²
Volume récepteur de 6.5 ml

### - Solution réceptrice :

Tampon Phosphate 10 mmol
NaCl 120 mmol
KCl 2,7 mmol
Azide de Sodium à 0.1 %
Tensio-actif : Tween 80^{®} à 0.5 %

### - Qualité des peaux :

Peaux d'oreilles de porcs non ébouillantées, ni tatouées, ni griffées, découpées en pièces de 3cm sur 3cm et tendues à la surface du compartiment inférieur des cellules de Franz.

### - Conditions d'application :

Dépôt randomisé sur chaque explant de 18 mg de formulation, soit 0.18 mg de principe actif (dose correspondante à une application en condition réelle d'utilisation d'un produit cosmétique).

Etude réalisée en condition occlusive sur peau totale.

### - Nombre de réplicates :

3 cellules de Franz pour chaque formule testée

### - Température et agitation :

Température moyenne du bain thermostaté à 34 °C
Agitation moyenne de 200 rpm

- *Appareil de prélèvement Rainin^{®}*
- *Pipette de prélèvement manuelle Rainin^{®}*

### - Conditions de dosages HPLC :

| | |
|---|---|
| *Appareil de dosage :* | Agilent^{®}, CD 017070 |
| *Colonne :* | Colonne HPLC RP18 greffée C18 endcapped, colonne apolaire en phase inverse Lichrospher^{®} 100 RP endcapped (125*4) mm, 5µm |
| *Eluant :* | A= H₂O + 0.1% HClO₄ |
| | B= Acétonitrile |
| *Gradient :* | Isocratique 15% de B |
| *Injection :* | 20 µl toutes les 20 min |
| *Détection :* | λ_{caféine}= 270 nm |
| *Débit :* | 1 ml/min |

### - Gamme étalon : mise en solution des étalons de 0.5 à 500 ppm dans un mélange eau/éthanol (50/50).

### - Concentration en caféine

Afin d'éviter de sous-estimer l'absorption cutanée, la concentration de caféine dans le liquide récepteur devait être inférieur à 10% de la concentration limite de solubilité.

La concentration limite de solubilité de la caféine dans le liquide récepteur était de 16 mg/ml. La concentration maximum de caféine recueillie au cours de l'étude était inférieure à 9.9 µg/ml.

### Résultats

Les résultats de cinétique de diffusion transmembranaire ont été exprimés sous forme d'histogrammes montrant ce rendement à 4H, 24H, 32H et 46H pour les deux formules sur la figure 2. La formule 1 correspond à la composition de l'exemple 1 selon l'invention, la formule 2 à celle de la formule témoin sans vésicule décrite à l'exemple comparatif 2.4. Les barres d'histogrammes expriment le rendement à différents temps, de la caféine ayant pénétré à travers la peau pour les deux formulations.

A chaque prélèvement, on a observé un passage transcutané plus important de la caféine dans le cas où la composition appliquée sur la peau comprend des vésicules à base de polypentosides d'alkyle tels que définis précédemment.

## Revendications

1. Composition cosmétique ou dermatologique comprenant au moins un agent actif cosmétique ou dermatologique et au moins une phase aqueuse continue dans laquelle sont dispersées des vésicules,
- lesdites vésicules comprenant au moins un polypentoside d'alkyle obtenu par réaction d'un pentose et d'au moins un premier et un deuxième alcools gras comprenant indépendamment l'un de l'autre un nombre d'atomes de carbone allant de 8 à 12, chacun de ces alcools gras pouvant être saturé ou insaturé, linéaire ou ramifié, ledit polypentoside ayant un degré de polymérisation moyen inférieur ou égal à 2, et
- ladite composition comprenant en outre au moins un tensioactif de HLB inférieur à 10, et au moins un alcool ayant de 1 à 5 atomes de carbone.

2. Composition selon la revendication 1, **caractérisée en ce que** le pentose est le D-xylose.

3. Composition selon l'une des revendications précédentes, **caractérisée en ce que** le degré de polymérisation moyen du polypentoside est compris entre 1 et 1,8.

4. Composition selon l'une des revendications précédentes, **caractérisée en ce que** le premier alcool gras est linéaire saturé et comprend de 10 à 12 atomes de carbone, et/ou le deuxième alcool gras est linéaire saturé et comprend 8 ou 9 atomes de carbone.

5. Composition selon la revendication précédente, **caractérisée en ce que** le premier alcool gras est le décanol-1 et/ou le deuxième alcool gras est l'octanol-1.

6. Composition selon l'une des revendications précédentes, **caractérisée en ce que** le rapport massique entre le premier et le deuxième alcool gras est compris entre 80/20 et 99/1.

7. Composition selon l'une des revendications précédentes, **caractérisée en ce qu'**elle comprend de 0,01 à 20% en poids total de polypentoside d'alkyle.

8. Composition selon l'une des revendications précédentes, **caractérisée en ce que** l'alcool est un mono-alcool choisi parmi l'éthanol, le butanol-1 et le pentanol-1, ou bien un diol choisi parmi le 2,3-butylène glycol ou le propane-1,2-diol.

9. Composition selon l'une des revendications précédentes, **caractérisée en ce que** le tensioactif de HLB inférieur à 10 est non ionique et choisi parmi les esters de sorbitan et d'un acide gras aliphatique non hydroxylé, et les esters de sorbitan faiblement éthoxylés.

10. Composition selon l'une des revendications précédentes, **caractérisée en ce que** le tensioactif de HLB inférieur à 10 est choisi parmi le monolaurate de sorbitan, le mono-oléate de sorbitan, le mono-stéarate de sorbitan, le tri-oléate de sorbitan, le tri-stéarate de sorbitan, le sesquioléate de sorbitan, et le POE(2) stéarate de sorbitan.

11. Composition selon l'une des revendications précédentes, **caractérisée en ce qu'**elle comprend du monolaurate de sorbitan, de l'éthanol et un polypentoside d'alkyle obtenu par réaction du D-xylose, du 1-décanol et du 1-octanol, ledit polypentoside ayant un degré de polymérisation moyen compris allant de 1,4 et 1,6.

12. Composition selon l'une des revendications précédentes, **caractérisée en ce qu'**elle comprend un agent actif cosmétique ou dermatologique qui est au moins partiellement encapsulé dans les vésicules.

13. Composition selon l'une des revendications précédentes, **caractérisée en ce qu'**elle est sous la forme d'une solution aqueuse ou hydro-alcoolique, d'une dispersion, d'un gel aqueux ou hydro-alcoolique, ou encore d'une émulsion huile dans eau (H/E).

14. Composition selon l'une des revendications précédentes, **caractérisée en ce qu'**elle comprend en outre au moins un gélifiant de la phase aqueuse choisi parmi les copolymères réticulés de l'acide acrylique et de l'acrylate d'éthyle, et les dérivés de cellulose.

15. Procédé de préparation de la composition selon l'une des revendications 1 à 14 comprenant les étapes suivantes :
- la préparation d'une phase aqueuse comprenant avantageusement l'agent actif cosmétique ou dermatologique, suivie de
- la dispersion du polypentoside d'alkyle dans ladite phase aqueuse, en apportant de l'énergie notamment sous la forme d'un cisaillement permettant la formation de vésicules à la température ambiante ou à une température supérieure mais de préférence inférieure à 100°C, de préférence inférieure à 80°C,
ledit procédé étant **caractérisé en ce que** l'alcool et l'agent tensioactif d'HLB inférieur à 10 sont chacun ajoutés partiellement ou en totalité, indifféremment soit dans la phase aqueuse, soit en mélange avec le polypentoside d'alkyle pour être ensuite dispersés dans ladite phase aqueuse.

## Patentansprüche

1. Kosmetische oder dermatologische Zusammensetzung, umfassend mindestens einen kosmetischen oder dermatologischen Wirkstoff und mindestens eine kontinuierliche wässrige Phase, in der Bläschen dispergiert sind,
- wobei die Bläschen mindestens ein Alkylpolypentosid umfassen, das durch Reaktion einer Pentose und mindestens eines ersten und eines zweiten Fettalkohols, umfassend unabhängig voneinander eine Anzahl von Kohlenstoffatomen von 8 bis 12, erhalten wird, wobei jeder dieser Fettalkohole gesättigt oder ungesättigt, linear oder verzweigt sein kann, wobei das Polypentosid einen mittleren Polymerisationsgrad kleiner oder gleich 2 aufwiest, und
- wobei die Zusammensetzung ferner mindestens ein Tensid mit einem HLB-Wert unter 10 und mindestens einen Alkohol mit 1 bis 5 Kohlenstoffatomen umfasst.

2. Zusammensetzung gemäß Anspruch 1, **dadurch gekennzeichnet, dass** die Pentose die D-Xylose ist.

3. Zusammensetzung gemäß einem der vorhergehenden Ansprüche, **dadurch gekennzeichnet, dass** der mittlere Polymerisationsgrad des Polypentosids zwischen 1 und 1,8 beträgt.

4. Zusammensetzung gemäß einem der vorhergehenden Ansprüche, **dadurch gekennzeichnet, dass** der erste Fettalkohol linear gesättigt ist und 10 bis 12 Kohlenstoffatome umfasst, und/oder der zweite Fettalkohol linear gesättigt ist und 8 oder 9 Kohlenstoffatome umfasst.

5. Zusammensetzung gemäß dem vorhergehenden Anspruch, **dadurch gekennzeichnet, dass** der erste Fettalkohol Decanol-1 und/oder der zweite Fettalkohol Octanol-1 ist.

6. Zusammensetzung gemäß einem der vorhergehenden Ansprüche, **dadurch gekennzeichnet, dass** das Massenverhältnis zwischen dem ersten und dem zweiten Fettalkohol zwischen 80/20 und 99/1 beträgt.

7. Zusammensetzung gemäß einem der vorhergehenden Ansprüche, **dadurch gekennzeichnet, dass** sie 0,01 bis 20 Gesamtgewichts-% Alkylpolypentosid umfasst.

8. Zusammensetzung gemäß einem der vorhergehenden Ansprüche, **dadurch gekennzeichnet, dass** der Alkohol ein Monoalkohol ist, ausgewählt unter Ethanol, Butanol-1 und Pentanol-1, oder auch ein Diol, ausgewählt unter 2,3-Butylenglykol oder Propan-1,2-diol.

9. Zusammensetzung gemäß einem der vorhergehenden Ansprüche, **dadurch gekennzeichnet, dass** das Tensid mit einem HLB-Wert unter 10 nicht ionisch ist und unter den Sorbitan-Estern und einer nicht hydroxylierten aliphatischen Fettsäure und den gering ethoxylierten Sorbitanestern ausgewählt ist.

10. Zusammensetzung gemäß einem der vorhergehenden Ansprüche, **dadurch gekennzeichnet, dass** das Tensid mit einem HLB-Wert unter 10 unter Sorbitan-Monolaurat, Sorbitan-Mono-Oleat, Sorbitan-Mono-Stearat, Sorbitan-Tri-Oleat, Sorbitan-Tri-Stearat, Sorbitan-Sesquioleat und Sorbitan-POE(2)Stearat ausgewählt ist.

11. Zusammensetzung gemäß einem der vorhergehenden Ansprüche, **dadurch gekennzeichnet, dass** sie Sorbitan-Monolaurat, Ethanol und Alkyl-Polypentosid, erhalten durch Reaktion der D-Xylose, des 1-Decanols und des 1-Octanols, umfasst, wobei das Polypentosid einen mittleren Polymerisationsgrad zwischen 1,4 und 1,6 aufweist.

12. Zusammensetzung gemäß einem der vorhergehenden Ansprüche, **dadurch gekennzeichnet, dass** sie einen kosmetischen oder dermatologischen Wirkstoff umfasst, der zumindest teilweise in den Bläschen verkapselt ist.

13. Zusammensetzung gemäß einem der vorhergehenden Ansprüche, **dadurch gekennzeichnet, dass** sie in Form einer wässrigen oder hydroalkoholischen Lösung, einer Dispersion, eines wässrigen oder hydroalkoholischen Gels oder auch einer Emulsion Öl in Wasser (H/E) vorhanden ist.

14. Zusammensetzung gemäß einem der vorhergehenden Ansprüche, **dadurch gekennzeichnet, dass** sie ferner mindestens ein Gelierungsmittel der wässrigen Phase umfasst, ausgewählt unter den vernetzten Copolymeren der Acrylsäure und des Ethylacrylats und den Zellulosederivaten.

15. Verfahren zur Herstellung der Zusammensetzung gemäß einem der Ansprüche 1 bis 14, umfassend die folgenden Schritte:
- Herstellung einer wässrigen Phase, umfassend vorteilhafterweise den kosmetischen oder dermatologischen Wirkstoff, gefolgt von
- der Dispersion des Alkylpolypentosids in der wässrigen Phase, wobei Energie insbesondere in Form einer Scherbelastung zugeführt wird, die die Bildung von Bläschen bei Raumtemperatur oder einer Temperatur über aber vorzugsweise unter 100 °C, vorzugsweise unter 80 °C, ermöglicht,
wobei das Verfahren **dadurch gekennzeichnet ist, dass** der Alkohol und das Tensid mit einem HLB-Wert unter 10 jeweils teilweise oder zur Gänze unterschiedslos entweder in der wässrigen Phase oder im Gemisch mit dem Alkylpolypentosid zugeführt werden, um sodann in der wässrigen Phase zerstreut zu werden.

## Claims

1. A cosmetic or dermatological composition comprising at least one cosmetic or dermatological active agent and at least one continuous aqueous phase in which are dispersed vesicles,
- said vesicles comprising at least one alkyl polypentoside obtained by reaction of a pentose and of at least a first and a second fatty alcohol comprising, independently of each other, a number of carbon atoms ranging from 8 to 12, each of these fatty alcohols being eventually saturated or unsaturated, and linear or branched, said polypentoside having an average polymerization degree of less than or equal to 2, and
- said composition also comprising at least one surfactant having a HLB of less than 10, and at least one alcohol containing from 1 to 5 carbon atoms.

2. The composition as claimed in claim 1, **characterized in that** the pentose is D-xylose.

3. The composition as claimed in either of the preceding claims, **characterized in that** the average polymerization degree of the polypentoside is between 1 and 1.8.

4. The composition as claimed in one of the preceding claims, **characterized in that** the first fatty alcohol is linear and saturated and comprises from 10 to 12 carbon atoms, and/or the second fatty alcohol is linear and saturated and comprises 8 or 9 carbon atoms.

5. The composition as claimed in the preceding claim, **characterized in that** the first fatty alcohol is 1-decanol and/or the second fatty alcohol is 1-octanol.

6. The composition as claimed in one of the preceding claims, **characterized in that** the mass ratio between the first and the second fatty alcohol is between 80/20 and 99/1.

7. The composition as claimed in one of the preceding claims, **characterized in that** it comprises from 0.01% to 20% by total weight of alkyl polypentoside.

8. The composition as claimed in one of the preceding claims, **characterized in that** the alcohol is a monoalcohol chosen from ethanol, 1-butanol and 1-pentanol, or a diol chosen from 2,3-butylene glycol and 1,2-propanediol.

9. The composition as claimed in one of the preceding claims, **characterized in that** the surfactant having a HLB of less than 10 is nonionic and chosen from sorbitan esters of a non-hydroxylated aliphatic fatty acid, and weakly ethoxylated sorbitan esters.

10. The composition as claimed in one of the preceding claims, **characterized in that** the surfactant having a HLB of less than 10 is chosen from sorbitan monolaurate, sorbitan monooleate, sorbitan monostearate, sorbitan trioleate, sorbitan tristearate, sorbitan sesquioleate and sorbitan POE(2) stearate.

11. The composition as claimed in one of the preceding claims, **characterized in that** it comprises sorbitan monolaurate, ethanol and an alkyl polypentoside obtained by reaction of D-xylose, 1-decanol and 1-octanol, said polypentoside having an average polymerization degree ranging from 1.4 to 1.6.

12. The composition as claimed in one of the preceding claims, **characterized in that** it comprises a cosmetic or dermatological active agent which is at least partially encapsulated in the vesicles.

13. The composition as claimed in one of the preceding claims, **characterized in that** it is in the form of an aqueous or aqueous-alcoholic solution, a dispersion, an aqueous or aqueous-alcoholic gel, or alternatively an oil-in-water (O/W) emulsion.

14. The composition as claimed in one of the preceding claims, **characterized in that** it also comprises at least one gelling agent for the aqueous phase chosen from crosslinked copolymers of acrylic acid and of ethyl acrylate, and cellulose derivatives.

15. A process for preparing the composition as claimed in one of claims 1 to 14, comprising the following steps:
- the preparation of an aqueous phase advantageously comprising the cosmetic or dermatological active agent, followed by
- the dispersion of the alkyl polypentoside in said aqueous phase, by providing energy especially in the form of a shear allowing the formation of vesicles at room temperature or at a higher temperature but preferably below 100°C and preferably below 80°C,
said process being **characterized in that** the alcohol and the surfactant having a HLB of less than 10 are each added, partially or totally, without preference either to the aqueous phase, or as a mixture with the alkyl polypentoside in order to be subsequently dispersed in said aqueous phase.
